# EUROPÄISCHE PATENTSCHRIFT

(11) **EP 0 817 612 B1**
(45) Veröffentlichungstag und Bekanntmachung des Hinweises auf die Patenterteilung: **06.09.2000**
(21) Anmeldenummer: 96907414.5
(22) Anmeldetag: 09.03.1996
(51) Int. Cl.: A61K 9/00, A61K 9/20

(54) **TRANSPARENTE, SCHNELL FREISETZENDE ZUBEREITUNGEN VON NICHTSTEROIDALEN ANALGETICA**
TRANSPARENT RAPID-RELEASE PREPARATIONS OF NON-STEROID ANALGESICS
PREPARATIONS TRANSPARENTES A LIBERATION RAPIDE D'ANALGESIQUES NON STEROIDES

(30) Priorität: 21.03.1995 DE 19509805
(43) Veröffentlichungstag der Anmeldung: 14.01.1998
(73) Patentinhaber: BASF AKTIENGESELLSCHAFT, 67056 Ludwigshafen (DE)
(72) Erfinder: BREITENBACH, Jörg, 68199 Mannheim (DE); SANNER, Axel, 67227 Frankenthal (DE); ROSENBERG, Joerg, 67158 Ellerstadt (DE)
(86) Internationale Anmeldenummer: EP9601021
(87) Internationale Veröffentlichungsnummer: WO9629053

(56) Entgegenhaltungen:
- EP-A- 0 358 107

## Beschreibung

Die vorliegende Erfindung betrifft transparente, schnellfreisetzende Zubereitungen nichtsteroidaler Analgetica mit antipyretischer und antiphlogistischer Wirkung, erhältlich durch Extrusion einer Schmelze, enthaltend neben einem oder mehreren Wirkstoffen
a) 50 bis 100 Gew.-% Homopolymeren des N-Vinylpyrrolidons mit einem K-Wert nach Fikentscher von 30,
b) 0 bis 30 Gew.-% an wasserlöslichen Sacchariden oder Zuckeralkoholen oder Mischungen daraus, und
c) 0 bis 20 Gew.-% an einem oder mehreren physiologisch akzeptabler Salze des Natriums oder des Kaliums,
wobei die Mengenangaben sich auf die Summe von a), b) und c) beziehen, und anschließender Formgebung.

Weiterhin betrifft die Erfindung ein Verfahren zur Herstellung solcher Zubereitungen.

Gerade bei Analgetica ist die schnelle Freisetzung des Wirkstoffs zur Erzielung einer rasch einsetzenden schmerzstillenden Wirkung von entscheidender Bedeutung.

Im Falle von schlecht wasserlöslichen Wirkstoffen wie sie beispielsweise die analgetisch wirksamen organischen Säuren darstellen, ist eine schnelle Freisetzung ausreichender Dosen oft nicht so einfach zu erreichen.

In der EP-A 607 467 wird vorgeschlagen, die schnelle Freisetzung von Ibuprofen durch einen Zusatz von basischen Salzen zu fördern, welche während des Pelletiervorgangs in Form wäßriger Lösungen auf den mit einem Hilfsstoff vorgemischten Wirkstoff aufgebracht werden. Die Pellets werden anschließend auf herkömmliche Weise zu Tabletten verpreßt. Diese Vorgehensweise ist jedoch relativ aufwendig und deshalb ökonomisch wenig günstig.

Es ist weiterhin bekannt, daß durch die Extrusion wirkstoffhaltiger Polymerschmelzen mit anschließender kontinuierlicher Formgebung die Herstellung von Arzneiformen auf sehr wirtschaftliche Weise erfolgen kann.

In der EP-B 240 904 wird ein solches Verfahren zur Herstellung fester pharmazeutischer Formen durch Extrusion wirkstoffhaltiger Polymerschmelzen beschrieben, wobei als Polymere Homo- oder Copolymere des N-Vinylpyrrolidons verwendet werden.

Bei einem solchen Verfahren besteht jedoch das grundsätzliche Problem, daß die matrixbildenden Polymere zum einen bei den Verarbeitungstemperaturen ausreichend thermoplastisch verarbeitbar sind bzw. durch Zusatz einer weichmachenden Substanz verarbeitbar werden, zum anderen aber unter den üblichen Lagerungsbedingungen zu stabilen Arzneiformen führen, bei denen kein "kalter Fluß" eintritt.

Dieses Problem ist umso schwerer lösbar, wenn man schnell freisetzende Arzneiformen herstellen will. Dazu eignen sich normalerweise vor allem relativ niedermolekulare Polymere, die sich in Verdauungssäften schnell auflösen. Gerade diese zeigen aber verstärkt das Phänomen des "kalten Flusses" bei den fertigen Arzneiformen. Höhermolekulare Polymere sind üblicherweise nicht schnell freisetzend und können ohne Weichmacher kaum extrudiert werden, da die Glastemperatur (DIN 52324) wesentlich höher liegt.

Ein zusätzliches Problem stellt sich, wenn man transparente Arzneiformen durch Schmelzextrusion herstellen will. Nur in transparenten Formen ist der Wirkstoff völlig gleichmäßig verteilt, ohne daß Kompartimentierung auftritt. Dies ist für eine schnelle Freisetzung unerläßlich. Zudem vereinfacht der Einsatz transparenter Formen die Qualitätskontrolle und die Patienten-Compliance.

Aufgabe der vorliegenden Erfindung war es transparente, schnell freisetzende Zubereitungen nichtsteroidaler Analgetica zu finden, die auf einfache Weise durch Schmelzextrusion mit anschließender Formgebung herstellbar sind und eine gute Lagerstabilität aufweisen.

Demgemäß wurden die eingangs definierten Zubereitungen gefunden.

Als Wirkstoffe kommen erfindungsgemäß nichtsteroidale Analgetica mit antipyretischer und antiphlogistischer Wirkung in Betracht, wie sie auch für die symptomatische antirheumatische Therapie eingesetzt werden.

Geeignete Wirkstoffe sind demgemäß Derivate der Salicylsäure wie Acetylsalicylsäure sowie Derivate anderer organischer Säuren und Pyrazolderivate. So kommen als Wirkstoffe Arylsäurederivate wie Diclofenac, Tolmetin oder Zomepirac in Betracht, weiterhin Arylpropylsäurederivate wie Ibuprofen, Naproxen, Fenoprofen, Flurbiprofen oder Ketoprofen oder auch Indol- und Indenessigsäurederivate wie beispielsweise Indometacin oder Sulindac. Geeignete Pyrazolderivate sind beispielsweise Phenazon, Aminophenazon, Metamizol, Propyphenazon, Phenylbutazon oder Oxyphenbutazon.

Bevorzugte Wirkstoffe sind Ibuprofen, Acetylsalicylsäure und Ketoprofen, Gulindac, Indometacin, Flurbiprofen.

Es können auch Wirkstoffgemische eingesetzt werden.

Als Komponenten a) enthalten die erfindungsgemäßen Zubereitungen ein Homopolymeres des N-Vinylpyrrolidons mit einem K-Wert nach Fikentscher von 30 (zur Definition des K-Wertes siehe "H. Fikentscher, Cellulose-Chemie", 13 (1932), S. 58-64 und 71-74). Dieses Homopolymere ist gut thermo- und wasserlöslich, wobei "wasserlöslich" bedeutet, daß sich in 100 g Wasser bei 20°C mindestens 0,5 g, vorzugsweise mindestens 2 g des Polymeren lösen, gegebenenfalls auch kolloidal. Die Herstellung des Homopolymeren ist allgemein bekannt.

Als Komponenten b) kommen wasserlösliche Saccharide oder Zuckeralkohole oder deren Mischungen in Betracht. Geeignete Saccharide sind vor allem Mono- oder Disaccharide wie Galactose, Fructose, Dextrose, Mannose, Maltose, Isomaltulose (Palatinose), Lactose oder Saccharose.

Geeignete Zuckeralkohole sind beispielsweise Mannit, Xylit, Sorbit, Adonit, Dulcit, generell Pentite und Hexite.

Als Komponenten c) kommen physiologisch verträgliche Natrium- und/oder Kaliumsalze in Betracht beispielsweise Natriumacetat, Kaliumacetat, Natriumcarbonat, Kaliumcarbonat, Dinatriumhydrogencarbonat, Natriumhydroxid, Kaliumhydroxid, Natriumchlorid oder Kaliumchlorid, wobei Natriumacetat bevorzugt ist.

Die Mengenverhältnisse der Komponente a), b) und c) werden erfindungsgemäß so gewählt, daß die Zubereitungen
a) 50 bis 100 Gew.-%, bevorzugt, 60 bis 90 Gew.-% der Komponente a),
b) 0 bis 30 Gew.-%, bevorzugt 5 bis 20 Gew.-%, der Komponente b), und
c) 0 bis 20 Gew.-%, bevorzugt 5 bis 20 Gew.-% der Komponente c) enthalten
wobei die Mengenangabe sich auf die Summe von a), b) und c) beziehen.

Bevorzugte Zubereitungen enthalten 80 bis 95 Gew.-% an Komponente a) und 5 bis 20 Gew.-% an Komponente c), bezogen auf die Summe von a) und c).

Die Gesamtzusammensetzung der Wirkstoffzubereitungen kann je nach erforderlicher Wirkdosis und Freisetzungsgeschwindigkeit in weiten Grenzen variiert werden. So kann der Anteil an Wirkstoff 0,1 bis 90, vorzugsweise 0,5 bis 60 Gew.-% der gesamten Zubereitung betragen.

Die erfindungsgemäßen Zubereitungen können zusätzlich übliche pharmazeutische Hilfsstoffe in den üblichen Mengen enthalten.

Das Mischen des Wirkstoffs oder der Wirkstoffe mit den polymeren Bindemitteln und gegebenenfalls galenischen Zusätzen kann vor oder nach dem Schmelzen des polymeren Bindemittels nach den in der Technik üblichen Verfahren erfolgen. Bevorzugt wird das Mischen im Extruder, vorzugsweise einem Zweischneckenextruder oder einem Einschneckenextruder mit Mischabteil.

Die Schmelzen sind lösungsmittelfrei. Damit ist gemeint, daß kein Wasser und kein organisches Lösungsmittel zugesetzt wird.

Die Herstellung erfolgt durch Extrusion bei 50 bis 180°C, vorzugsweise 60 bis 150°C und anschließende Verformung des noch plastischen Stranges, z.B. durch Verformen zu Tabletten, beispielsweise gemäß EP-A 240 906 durch Hindurchführen des Stranges zwischen zwei gegenläufig angetriebenen Walzen mit einander gegenüberliegenden Vertiefungen im Walzenmantel, deren Ausführung die Tablettenform bestimmt. Auch Kaltabschlag kommt in Betracht.

Bevorzugt wird der sogenannte Heißabschlag. Dabei werden die Stränge unmittelbar nach dem Austritt aus der Düsenanordnung am Extruder durch beispielsweise rotierende Messer oder eine andere geeignete Anordnung zerkleinert, zweckmäßig in Stücke, deren Länge etwa gleich dem Strangdurchmesser ist. Diese abgeschlagenen Schmelzteilchen kühlen im Luft- oder Gasstrom so weit ab, daß die Oberfläche vor einer Berührung mit anderen Teilchen oder einer Gefäßwand bereits klebfrei ist, andererseits die Teilchen aber noch so plastisch sind, daß sie durch Zusammenstöße, z.B. mit der Wandung eines angeschlossenen Cyclons, eine sphärische Form erhalten. Man erhält so in einfacher Weise weitgehend kugel- oder linsenförmige Teilchen mit Durchmessern von 0,5 bis 4, vorzugsweise 0,8 bis 2 mm. Die bevorzugten kleineren Teilchen sind in erster Linie zum Füllen von Kapseln geeignet.

Die festen Arzneiformen können auch mit einem üblichen Überzug zur Verbesserung des Aussehens und/oder des Geschmacks (Dragee) versehen werden.

Die erfindungsgemäßen Zubereitungen von nichtsteroidalen Analgetica mit antipyretischer und antiphlogistischer Wirkung sind transparent, lagerstabil und schnell freisetzend. "Schnell freisetzend" bedeutet, daß die Freisetzung des Wirkstoffs nach 30 min, gemessen nach der Paddle-Methode nach USP XXII, mindestens 70 % beträgt.

Überraschenderweise waren trotz Verwendung eines relativ hochmolekularen Polymers sogar Arzneiformen mit hohen Gewichten wie 1000 mg schnell freisetzend. Vorteilhaft an großen Tabletten ist auch, daß sie als Lutschtabletten angewendet werden können, ohne verschluckt zu werden, wobei durch den Zusatz von Zuckeralkoholen auch keine Geschmacksprobleme auftreten. Gerade bei älteren Patienten oder bei Patienten mit Dysphagie ist das Schlucken größerer Tabletten of mit Schwierigkeiten verbunden, so daß schnell freisetzende Lutschtabletten große Vorzüge aufweisen.

### Beispiele

Die in den Beispielen jeweils angegebenen Zusammensetzungen wurden vorgemischt und in den Einzug eines Doppelschnecken-Extruders (Werner & Pfleiderer, ZSK 30) eingetragen. Die Schmelzextrusion erfolgte mit einem Produktdurchsatz von 3 bis 4 kg/h. Die Temperaturen der einzelnen Temperaturzonen ("Schüsse") des Extruders sowie die Temperatur der beheizten Düsenleiste ist bei den Versuchen jeweils angegeben. Aus dem Extrudat wurden gemäß dem in der EP-B 240 906 beschriebenen Kalandrierverfahren Bolus-Tabletten mit einem Gewicht von 1000 mg hergestellt.

Die Wirkstofffreisetzung wurde mittels der Rührflügelmethode (Paddle-Methode nach USP XXIII, US-Arzneibuch) gemessen. Diese invitro-Prüfungsmethode dient zur Bestimmung der Lösungsrate von wirkstoffhaltigen Formlingen, z.B. Tabletten.

Hierzu wurden 900 ml eines Phosphatpuffers mit einem pH-Wert von 6,8 mit einem Zusatz von 0,1 % Natriumlaurylsulfat in einem 1 L-Gefäß mit Rundboden auf 37°C temperiert. Eine entsprechende Menge an Arzneiform wurde eingewogen. Die Wirkstofffreisetzung der Boli wurde in diesem No-Change-Test nach USP XXI bei einer Paddle-Drehzahl von 100 Upm nach jeweils 10 Min. Stunden UV-spektroskopisch bestimmt.

### Beispiel 1

Temperaturen der Extruder-Zonen (Schüsse 1 - 5) 20, 80, 140, 130, 130°C, Temperatur Extruder-Kopf 130°C, Temperatur Düsenleiste 130°C

| Wirkstoff | Komponente a |
|---|---|
| Ibuprofen 20 Gew.-% | Polyvinylpyrrolidon K-Wert 30, 80 Gew.-% |

Freisetzung nach 30 Min. 82 %

### Beispiel 2

Temperaturen der Extruder-Zonen (Schüsse 1 - 5) 60, 120, 120, 110, 120°C, Temperatur Extruder-Kopf 130°C, Temperatur Düsenleiste 120°C

| Wirkstoff | Komponente a | Komponente b |
|---|---|---|
| Ibuprofen 20 Gew.-% | Polyvinylpyrrolidon K-Wert 30, 70 Gew.-% | D-Mannit 10 Gew.-% |

Freisetzung nach 30 Min. 72 %

### Beispiel 3

Temperaturen der Extruder-Zonen (Schüsse 1 - 5) 60, 120, 120, 120, 130°C, Temperatur Extruder-Kopf 130°C, Temperatur Düsenleiste 160°C

| Wirkstoff | Komponente a | Komponente b |
|---|---|---|
| Ibuprofen 20 Gew.-% | Polyvinylpyrrolidon K-Wert 30, 60 Gew.-% | D-Mannit 20 Gew.-% |

Freisetzung nach 30 Min. 70%

### Beispiel 4

Temperaturen der Extruder-Zonen (Schüsse 1 - 5) 70, 130, 130, 140, 130°C, Temperatur Extruder-Kopf 130°C, Temperatur Düsenleiste 160°C

| Wirkstoff | Komponente a | Komponente c |
|---|---|---|
| Ibuprofen 20 Gew.-% | Polyvinylpyrrolidon K-Wert 30, 66,5 Gew.-% | Na-Acetat 13,5 Gew.-% |

Freisetzung nach 30 Min. 95 %.

### Beispiel 5

Temperaturen der Extruder-Zonen (Schüsse 1 - 5) 70, 130, 130, 140, 130°C, Temperatur Extruder-Kopf 130°C, Temperatur Düsenleiste 160°C

| Wirkstoff | Komponente a | Komponente c |
|---|---|---|
| Ibuprofen 20 Gew.-% | Polyvinylpyrrolidon K-Wert 30, 75 Gew.-% | Na-Acetat 5 Gew.-% |

Freisetzung nach 30 Min. 95 %.

### Beispiel 6

Temperaturen der Extruder-Zonen (Schüsse 1 - 5) 60, 120, 120, 120, 130°C, Temperatur Extruder-Kopf 130°C, Temperatur Düsenleiste 160°C

| Wirkstoff | Komponente a | Komponente b | Komponente c |
|---|---|---|---|
| Ibuprofen 20 Gew.-% | Polyvinylpyrrolidon K-Wert 30, 70 Gew.-% | D-Mannit 5 Gew.-% | Na-Acetat 5 Gew.-% |

Freisetzung nach 30 Min. 80 %.

## Patentansprüche

1. Transparente, schnell freisetzende Zubereitungen nichtsteroidaler Analgetica, mit antipyretischer und antiphlogistischer Wirkung, erhältlich durch Extrusion einer Schmelze, enthaltend neben einem oder mehreren Wirkstoffen
a) 50 bis 100 Gew.-% Homopolymere des N-Vinylpyrrolidons mit einem K-Wert nach Fikentscher von 30,
b) 0 bis 30 Gew.-% an wasserlöslichen Sacchariden oder Zuckeralkoholen oder Mischungen daraus, und
c) 0 bis 20 Gew.-% an einem oder mehreren physiologisch akzeptablen Salzen des Natriums oder des Kaliums,
wobei die Mengenangaben sich auf die Summe von a), b) und c) beziehen,
und anschließender Formgebung.

2. Zubereitung nach Anspruch 1, enthaltend 5 bis 20 Gew.-% der Komponente c).

3. Zubereitungen nach Anspruch 1 oder 2, enthaltend als Wirkstoff Ibuprofen.

4. Zubereitungen nach einem der Ansprüche 1 bis 3 enthaltend als Komponente c) Natriumacetat.

5. Verfahren zur Herstellung transparenter, schnell freisetzender Zubereitungen gemäß einem der Ansprüche 1 bis 4, dadurch gekennzeichnet, daß man eine Schmelze, enthaltend neben einem oder mehreren Wirkstoffen
a) 50 bis 100 Gew.-% Homopolymere des N-Vinylpyrrolidons mit einem K-Wert nach Fikentscher von 30,
b) 0 bis 30 Gew.-% an wasserlöslichen Sacchariden oder Zuckeralkoholen oder Mischungen daraus, und
c) 0 bis 20 Gew.-% an einem oder mehreren physiologisch akzeptablen Salzen des Natriums oder des Kaliums,
wobei die Mengenangaben sich auf die Summe von a), b) und c) beziehen, bei 50 - 180°C extrudiert und anschließend verformt.

## Claims

1. A transparent rapid release composition of a non-steroidal analgesic with antipyretic and antiinflammatory action, obtainable by extrusion of a melt comprising, besides one or more active substances,
a) 50-100% by weight of homopolymers of N-vinylpyrrolidone with a Fikentscher K value of 30,
b) 0-30% by weight of water-soluble saccharides or sugar alcohols or mixtures thereof,
c) 0-20% by weight of one or more physiologically acceptable salts of sodium or of potassium,
where the stated amounts are based on the total of a), b) and c),
and subsequent shaping.

2. A composition as claimed in claim 1, comprising 5-20% by weight of component c).

3. A composition as claimed in claim 1 or 2, comprising ibuprofen as active substance.

4. A composition as claimed in any of claims 1 to 3, comprising sodium acetate as component c).

5. A process for producing transparent rapid release compositions as claimed in any of claims 1 to 4, which comprises extrusion of a melt comprising, besides one or more active substances,
a) 50-100% by weight of homopolymers of N-vinylpyrrolidone with a Fikentscher K value of 30,
b) 0-30% by weight of water-soluble saccharides or sugar alcohols or mixtures thereof,
c) 0-20% by weight of one or more physiologically acceptable salts of sodium or of potassium,
where the stated amounts are based on the total of a), b) and c), at 50-180°C, and subsequent shaping.

## Revendications

1. Compositions transparentes, à libération rapide, d'analgésiques non-stéroïques, ayant des activités antipyrétique et antiphlogistique, qu'on obtient par extrusion d'une masse fondue contenant, avec une ou plusieurs substances actives,
a) 50 à 100 % en poids d'homopolymères de la N-vinylpyrrolidone ayant un indice K selon Fikentscher de 30,
b) 0 à 30 % en poids de saccharides ou d'alcools des sucres, solubles dans l'eau, ou de leurs mélanges, et
c) 0 à 20 % en poids d'un ou plusieurs sels de sodium ou de potassium acceptables pour l'usage pharmaceutique,
les quantités indiquées se rapportant à la somme de a), b) et c), en faisant suivre d'un façonnage.

2. Composition selon la revendication 1, contenant 5 à 20 % en poids du composant c).

3. Compositions selon la revendication 1 ou 2, contenant l'Ibuprofen en tant que substance active.

4. Compositions selon une des revendications 1 à 3, contenant l'acétate de sodium en tant que composant c).

5. Procédé de préparation de compositions transparentes, à libération rapide, selon une des revendications 1 à 4, caractérisé par le fait que l'on extrude à une température de 50 à 180°C une masse fondue contenant, avec une ou plusieurs substances actives,
a) 50 à 100 % en poids d'homopolymères de la N-vinylpyrrolidone ayant un indice K selon Fikentscher de 30,
b) 0 à 30 % en poids de saccharides ou d'alcools des sucres, solubles dans l'eau, ou de leurs mélanges, et
c) 0 à 20 % en poids d'un ou plusieurs sels de sodium ou de potassium acceptables pour l'usage pharmaceutique,
les quantités indiquées se rapportant à la somme a), b) et c), puis on soumet à façonnage.
